# EUROPEAN PATENT SPECIFICATION

(11) **EP 0 371 503 B1**
(45) Date of publication and mention of the grant of the patent: **08.03.1995**
(21) Application number: 89122108.7
(22) Date of filing: 30.11.1989
(51) Int. Cl.: G01N 33/52, C12Q 1/00

(54) **Device for assay of liquid sample**
Vorrichtung zum Testen einer Flüssigkeitsprobe
Appareil d'essai d'un échantillon liquide

(30) Priority: 30.11.1988 JP 157143/88
(43) Date of publication of application: 06.06.1990
(73) Proprietor: KYOTO DAIICHI KAGAKU CO., LTD., Kyoto-shi Kyoto-fu (JP)
(72) Inventor: Sakamoto, Hisashi, Yawata-shi Kyoto-fu (JP); Yamada, Shigeki, Joyo-shi Kyoto-fu (JP); Taniguchi, Hiroshi, Fukakusa Fushimi-ku Kyoto-shi Kyoto-fu (JP)
(74) Representative: von Kreisler, Alek, Dipl.-Chem.

(56) References cited:
- EP-A- 256 806
- EP-A- 0 113 896
- EP-A- 0 114 403
- EP-A- 0 207 360
- EP-A- 0 272 407

## Description

The present invention relates to a device for colorimetric assay of a liquid sample, and more particularly, to a dry type device for colorimetric assay of at least one component in a liquid sample, for example, body fluids such as blood.

Assay of body fluids such as blood has been done in hospitals or by assay experts. Recently, with the development of dry type assay device, a patient can carry out the assay easily, quickly and precisely. For example, the patient samples a small amount of his or her blood and determines a glucose content in the blood so as to control food.

Typical examples of the dry type assay devices are disclosed in U.S. Patent Nos. 3,298,789 and 3,630,957 and Japanese Patent Publication No. 33800/1974. With the device of the U.S. Patent, the sampled whole blood is placed on a reagent pad covered with an ethylcellulose layer. After a predetermined period of time, the blood sample is washed out with water, and developed color of the reagent pad is detected. If erythrocytes remain on or in the reagent pad, they interfere detection of the developed color. To avoid such interference the cellulose layer is provided to prevent penetration of the erythrocytes into the reagent pad to facilitate removal of the erythrocytes. With the device of the above Japanese Patent Publication, a reagent pad comprises a water-resistant film which prevents penetration of the erythrocytes into the reagent pad.

Various special equipments have been developed and used in order to simplify control of time for assay and determination of a degree of developed color by the patient and also to increase reliability of data. With such equipments, the patient punctures a blood vessel and then he or she should start a time control means of the equipment at the same time as he or she applies a drop of blood on a reagent layer of the assay device such as an assay stick.

However, it requires some skill to start the time control means and to apply the drop of blood on the reagent layer simultaneously. In most cases, just after the application of the drop of blood on the reagent layer, the patient starts the time control means. Therefore, the reliability of the data is not satisfactory. Further, it is troublesome for the patient to apply the drop of blood and to start the time control means simultaneously. In addition, the patient should wipe out the blood after a predetermined period of time from the application of blood and then set the assay device in the measuring equipment. Such procedures are troublesome for the patient and deteriorate the reliability of the data.

Among the above problems, troublesome wiping of the blood can be neglected by the assay devices disclosed in U.S. Patent No. 3,992,158, DE-A-3 029 301, Japanese Patent Publication Nos. 21677/1978 and 164356/1980 and Japanese Patent Kokai Publication No. 24576/1981. For example, the device disclosed in U.S. Patent No. 3,992,158 is a multilayer integral assay element comprising a transparent support, a reagent layer and a porous spreading layer which are laminated successively. In use, when the sample is applied on a spot of the spreading layer, it spreads in a transverse direction and migrates into the reagent layer to develop the color in the reagent layer. Then, the degree of color development is observed through the transparent support. With such device, the color development is measured without the removal of the applied blood. However, such device cannot overcome such a problem that the control of time should be started simultaneously with the application of blood.

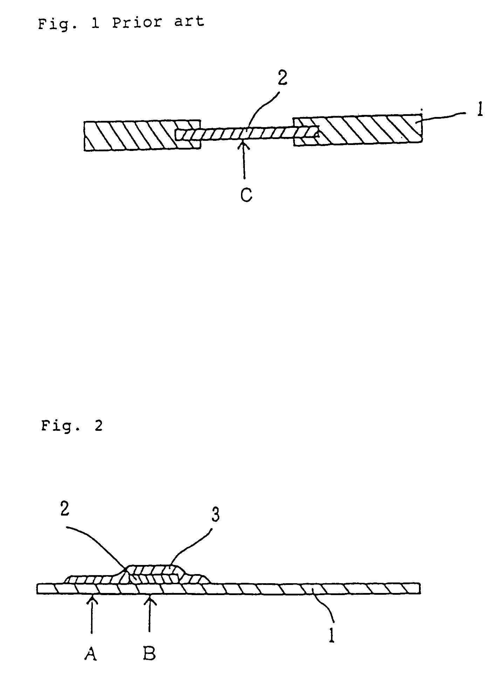

To avoid the troublesome time control, EP-A-256 806 and Japanese Patent Kokai Publication No. 101757/1988 discloses an equipment which can measure the blood color and the developed color with two beams of light having different wavelengths one of which is absorbed by the erythrocytes and the other of which by the developed color from an opposite side to the sample applied side. In measurement, a drop of blood is applied on the reagent layer of the dry type assay device as shown in Fig. 1 which is set in the measuring equipment. That is, when the drop of blood is applied on the reagent layer 2 supported by a support 1 of the dry type assay device, reflectance of the light having the wavelength which is absorbed by the erythrocytes decreases, and the start of such decrease of reflectance is automatically detected by the equipment and regarded as the application of blood on the dry type assay device.

However, this type of equipment has such a drawback that it tends to be influenced by stray light. That is, in this equipment, the application of sample and the degree of color development are both measured as changes of amounts of reflected lights at the same point indicated by the arrow C in Fig. 1. The timing of the sample application is detected as the change of amount of reflected light when the sample is applied. In general, the reflectance of light is expressed in terms of a percentage of the amount of light reflected on the reagent layer to the amount of light reflected on a standard white plate of magnesium oxide. In this equipment, since the amount of light from the reagent should be measured on the opposite side to the side which is illuminated with the measuring light, the reagent layer is transparent to some extent, and therefore, the amount of light received by light receiving means of the equipment is a sum of the light reflected by the reagent section and light from ambient light sources (e.g. the sun or room lamps) which passes through the reagent section and reaches the light receiving means. In measurement with this equipment, particularly when the room is light, as a finger reaches the reagent layer to apply the blood, the surface of reagent layer is shadowed with the finger. Then, even if the blood is not actually applied on the reagent layer, the reflectance is changed so that the timing of start of measurement may not be correctly detected. In addition, this equipment may have an insufficient dynamic range, since the application of the sample and the degree of color development are both measured by the measurement of reflectance on the reagent layer, so that the degree of color development is measured by using the reflectance of the reagent layer including the blood as a background. Since the blood absorbs not only light at a specific wavelength but also light from the developed color and further light outside the visible light range, when the reflected light is measured against the erythrocyte component as the background, the decrease of optical dynamic range cannot be avoided. The decrease of optical dynamic range decreases the accuracy of measurement. The transparency of the reagent layer which is necessary for the detection of application of the sample is one of the causes for the decrease of dynamic range. EP-A-207 360 discloses a test device comprising a support, a bibulous material placed on a part of said support containing a reagent system (reagent layer) and an analyte-permeable film which covers said reagent layer and said support (sample-receiving layer).

One object of the present invention is to provide an assay device which can determine the application timing of the sample on the device without malfunction.

Another object of the present invention is to provide an assay device which has a sufficient optical dynamic range.

Accordingly, the present invention provides a device for colorimetric assay of at least one component in a liquid sample as defined in claim 1. Further embodiments can be found in the dependent claims.
Fig. 1 is a cross section of one of the conventional assay devices,
Fig. 2 is a cross section of one embodiment of the assay device of the present invention, and
Fig. 3 to 7 are cross sections of various modifications of the assay device of the present invention.

A basic assay device of the present invention is shown in Fig. 2. This device comprises a support 1 made of, for example, a transparent plastic strip. On one surface of the support 1, a reagent layer 2 is directly formed, and a sample-receiving layer 3 is provided to cover the reagent layer 2 and a part of the surface of the support 1. The sample-receiving layer 3 does not necessarily cover the whole area of the reagent layer 2.

The reagent layer 2 contains at least one reagent which reacts with the component to be analyzed in the liquid sample to develop a specific color and a light reflecting agent which serves as a background.

Examples of the component to be analyzed are biochemical items in clinical tests such as glucose, galactose, urea nitrogen, uric acid, creatinine, ammonia, total proteins, albumin, lactic acid, glutamic-pyruvic transaminase, glutamic-oxaloacetic transaminase, alkaline phosphatase, lactate dehydrogenase, triglyceride, cholesterol, phospholipid, ketone body, bilirubin and hemoglobin.

The reagents which react with the respective components are well known. For example, in case of analysis of glucose, a combination of glucose oxidase, peroxidase and ortho-toluidine is used. Since ortho-toluidine turns blue in a degree corresponding to the amount of glucose, the degree of developed blue color is measured to determine the amount of glucose. When the ketone body is analyzed, as disclosed in Japanese Patent Publication No. 38199/1988, a combination of beta-hydroxybutyric dehydrogenase, nicotinamide adenine dinucleotide, diaphorase and phenadine methosulfate is used. With such combination, among the ketone body, beta-hydroxybutyric acid which is useful as a criteria for diabetes can be quantitatively measured.

Examples of the light-reflecting agent are metal oxides such as titanium dioxide, magnesium oxide and zinc oxide. The light-reflecting agent is useful to remove the interfering materials such as blood cells when the sample migrates from the sample-receiving layer to the reagent layer.

Since the sample-receiving layer covers a part of the support directly as shown in Fig. 2, it enables to detect the change of reflectance on said layer through only the support but not the reagent layer at a position of the support indicated by the arrow A in Fig. 2.

Since the timing of sample application is detected at a position apart from the reagent layer, the light can be sufficiently reflected at the reagent layer so that the sufficiently wide optical dynamic range can be achieved.

To receive the sample and spread it over the sample-receiving layer, the sample-receiving layer is preferably made of a hydrophilic porous material such as a filter paper, a fabric, a non-woven fabric and a mesh.

Several modifications of the assay device of the present invention can be contemplated.

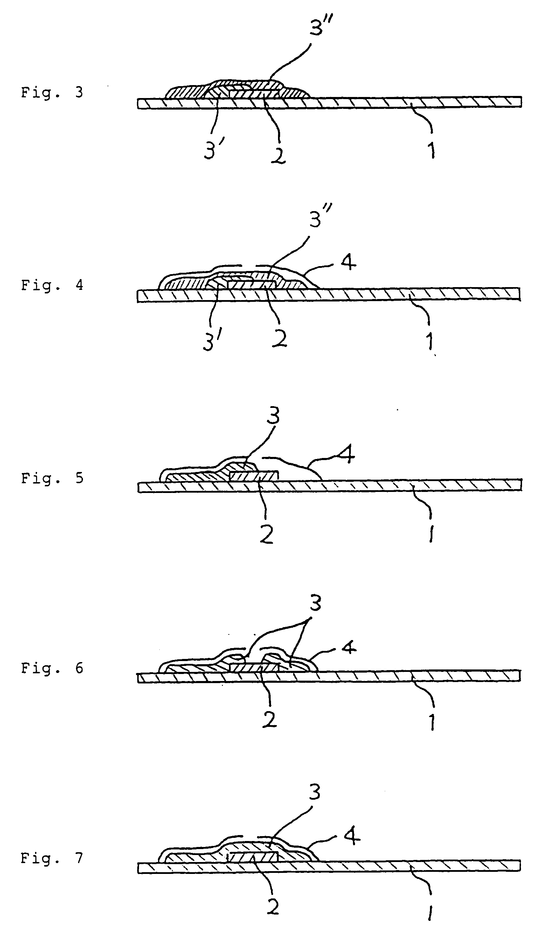

Fig. 3 is a cross section of the a modification. In this device, a part of the reagent layer 2 is covered with a porous material 3′ and then covered with a mesh layer 3˝ which covers the whole area of the reagent layer 2 and a part of the support 1.

Fig. 4 is a cross section of a further modification of the device of Fig. 3. In this device, a covering 4 is provided over the mesh layer 3˝.

Fig. 5 is a cross section of a second modification. In this device, the sample-receiving layer 3 covers a part of the reagent layer 2 and a part of the support, and a covering 4 is provided over the reagent layer 2 and the sample-receiving layer 3. The device of Fig. 5 can be further modified as shown in Fig. 6.

Fig. 7 is a modification of the basic device of Fig. 2 and has a covering 4.

How to carry out the measurement with the use of the assay device of the present invention will be illustrated.

As explained above, Fig. 2 shows a basic embodiment of the assay device of the present invention. In this device, the sample-receiving layer 3 covers the whole area of the reagent layer 2 and also a part of the support directly. The size of the thickness direction is enlarged for easy understanding.

In measurement, the assay device is set in the assay equipment. The equipment has two sets of reflectance measuring mechanisms, one of which measures directly the reflectance on the sample-receiving layer 3 at the position indicated by the arrow A and the other of which measures the reflectance on the reagent layer 3 at the position indicated by the arrow B.

When the sample is dropped on the sample-receiving layer 3, it spread over the whole area of said layer 3. Then, the reflectance over the position indicated by the arrow A changes, and such reflectance change is measured by the reflectance measuring mechanism to detect the application of sample. From such reflectance change, time is counted. After a predetermined period of time, the reflectance at the position indicated by the arrow B is measured. When the covering is provided over the reagent layer as shown in Figs. 4, 5, 6 and 7, the influence of stray light can be minimized. The covering may be made of a liquid-impermeable material. The measured reflectance is converted to the concentration of the particular component by using a calibration line which is beforehand produced.

## Claims

1. A device for colorimetric assay of at least one component in a liquid sample, which device comprises:
a transparent support,
a reagent layer formed on a part of one surface of the support and containing at least one reagent which reacts with a component in the sample to be analyzed to develop a specific color which is measured colorimetrically and a light reflecting agent, and
a sample-receiving layer which covers at least a part of the reagent layer and at least a part of the support surface wherein the timing of the sample application onto the device is accurately determined since the reflectance of the sample-receiving layer may be changed.

2. The device according to claim 1, wherein the sample-receiving layer covers the whole area of the reagent layer.

3. The device according to claim 1, which further comprises a covering which covers the reagent layer and the sample-receiving layer.

4. The device according to claim 2, which further comprises a covering which covers the sample-receiving layer.

## Patentansprüche

1. Vorrichtung zur kolorimetrischen Bestimmung wenigstens einer Komponente in einer flüssigen Probe, wobei die Vorrichtung umfaßt:
einen transparenten Träger,
eine Reagenzschicht, die auf einem Teil einer Oberfläche des Trägers ausgebildet ist und die wenigstens ein Reagenz enthält, das mit einer Komponente in der zu analysierenden Probe reagiert, um eine spezifische Farbe zu entwickeln, die kolorimetrisch gemessen wird, und ein Licht reflektierendes Mittel, und
eine probeaufnehmende Schicht, die wenigstens einen Teil der Reagenzschicht und wenigstens einen Teil der Trägeroberfläche bedeckt, wobei die Zeitbestimmung der Aufbringung der Probe auf die Vorrichtung genau bestimmt wird, da das Reflexionsvermögen der probeaufnehmenden Schicht verändert werden kann.

2. Vorrichtung nach Anspruch 1, wobei die probeaufnehmende Schicht die gesamte Fläche der Reagenzschicht bedeckt.

3. Vorrichtung nach Anspruch 1, die darüber hinaus einen Überzug umfasst, der die Reagenzschicht und die probeaufnehmende Schicht bedeckt.

4. Vorrichtung nach Anspruch 2, die darüber hinaus einen Überzug umfasst, der die probeaufnehmende Schicht bedeckt.

## Revendications

1. Dispositif d'analyse colorimétrique d'au moins un ingrédient dans un échantillon liquide, le dispositif comprenant :
un support transparent,
une couche réactive formée sur une partie d'une surface du support et contenant au moins un réactif qui réagit avec un ingrédient de l'échantillon à analyser avec création d'une couleur particulière qui est mesurée colorimétriquement, et un agent réfléchissant la lumière, et
une couche de réception d'échantillon qui recouvre une partie au moins de la couche réactive et une partie au moins de la surface de support, dans lequel le moment de l'application de l'échantillon sur le dispositif est déterminé avec précision puisque le pouvoir réflecteur de la couche de réception d'échantillon peut être modifié.

2. Dispositif selon la revendication 1, dans lequel la couche de réception d'échantillon recouvre toute la surface de la couche réactive.

3. Dispositif selon la revendication 1, qui comporte en outre un organe de recouvrement qui recouvre la couche réactive et la couche de réception d'échantillon.

4. Dispositif selon la revendication 2, qui comporte en outre un organe de recouvrement qui recouvre la couche réceptrice d'échantillon.
